(19) Europäisches Patentamt
European Patent Office
Office européen des brevets

(11) Veröffentlichungsnummer : **0 512 952 A1**

# (12) EUROPÄISCHE PATENTANMELDUNG

(21) Anmeldenummer : **92810306.8**

(22) Anmeldetag : **28.04.92**

(51) Int. Cl.[5] : **C07D 401/12,** A61K 31/44

(30) Priorität : **02.05.91 DE 4114325**

(43) Veröffentlichungstag der Anmeldung :
**11.11.92 Patentblatt 92/46**

(84) Benannte Vertragsstaaten :
**AT BE CH DE DK ES FR GB GR IT LI LU NL PT SE**

(71) Anmelder : **SANDOZ LTD.**
**Lichtstrasse 35**
**CH-4002 Basel (CH)**
(84) **BE CH DK ES FR GB GR IT LI LU NL PT SE**

(71) Anmelder : **SANDOZ-PATENT-GMBH**
**Humboldtstrasse 3**
**W-7850 Lörrach (DE)**
(84) **DE**

(71) Anmelder : **SANDOZ-ERFINDUNGEN Verwaltungsgesellschaft m.b.H.**
**Brunner Strasse 59**
**A-1235 Wien (AT)**
(84) **AT**

(72) Erfinder : **Gull, Peter**
**Moosackerweg 3**
**CH-4148 Pfeffingen (CH)**
Erfinder : **Markstein, Rudolf**
**Ernst-Reuter-Strasse 2**
**W-7888 Rheinfelden (DE)**

(54) **Octahydrobenzo(g)chinolin.**

(57) Die Erfindung betrifft die Verbindung der Formel

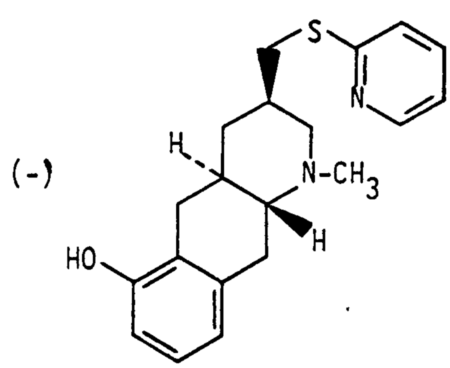

(I)

in Form der freien Base oder in Säureadditionssalzform, seine Herstellung und Anwendung als Arzneimittel.

EP 0 512 952 A1

Gegenstand der Erfindung ist ein neues Octahydrobenzo[g]chinolin, seine Herstellung und Anwendung bei der therapeutischen Behandlung.

Die Erfindung betrifft das (-)-(3β,4aα,10aβ)-1,2,3,4,4a,5,10,10a-Octahydro-3-[(2-pyridylthio)methyl]-1-methyl-6-hydroxy-benzo[g]chinolin der Formel I

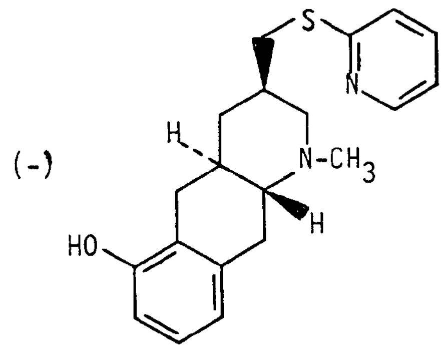

(I)

in Form der freien Base oder in Säureadditionssalzform.

Aus dem Europäischen Patent Nr. 77754 sind strukturell verwandte Verbindungen bekannt. Die Verbindung der Formel I ist jedoch nie spezifisch beschrieben worden. Es wurde nun überraschenderweise gefunden, dass diese Verbindung ein besonders interessantes pharmakologisches Wirkungsprofil aufweist.

Erfindungsgemäss gelangt man zu der Verbindung der Formel I und ihren Säureadditionssalzen, indem man die Verbindung der Formel II

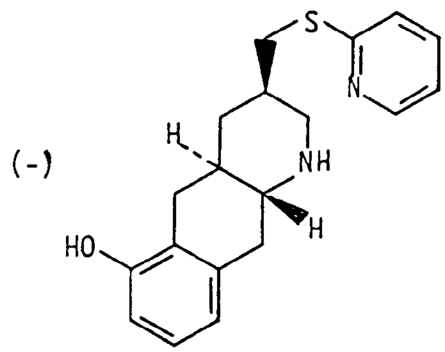

(II)

methyliert und die erhaltene Verbindung gewünschtenfalls in ihre Säureadditionssalze überführt.

Das erfindungsgemässe Verfahren kann nach an sich bekannten Methoden durchgeführt werden, beispielsweise mit Formaldehyd/$NaBH_4$ wie im nachfolgenden Beispiel unter e) beschrieben.

Die Aufarbeitung des erhaltenen Reaktionsgemisches und die Reinigung der so gewonnenen Verbindung der Formel I kann nach an sich bekannten Methoden durchgeführt werden.

Aus der freien Base lassen sich in bekannter Weise Säureadditionssalze herstellen und umgekehrt.

Die Ausgangsverbindung der Formel II kann ausgehend von der Verbindung der Formel VII nach dem folgenden Reaktionsschema, beispielsweise wie im Beispiel unter a) bis d) beschrieben, hergestellt werden:

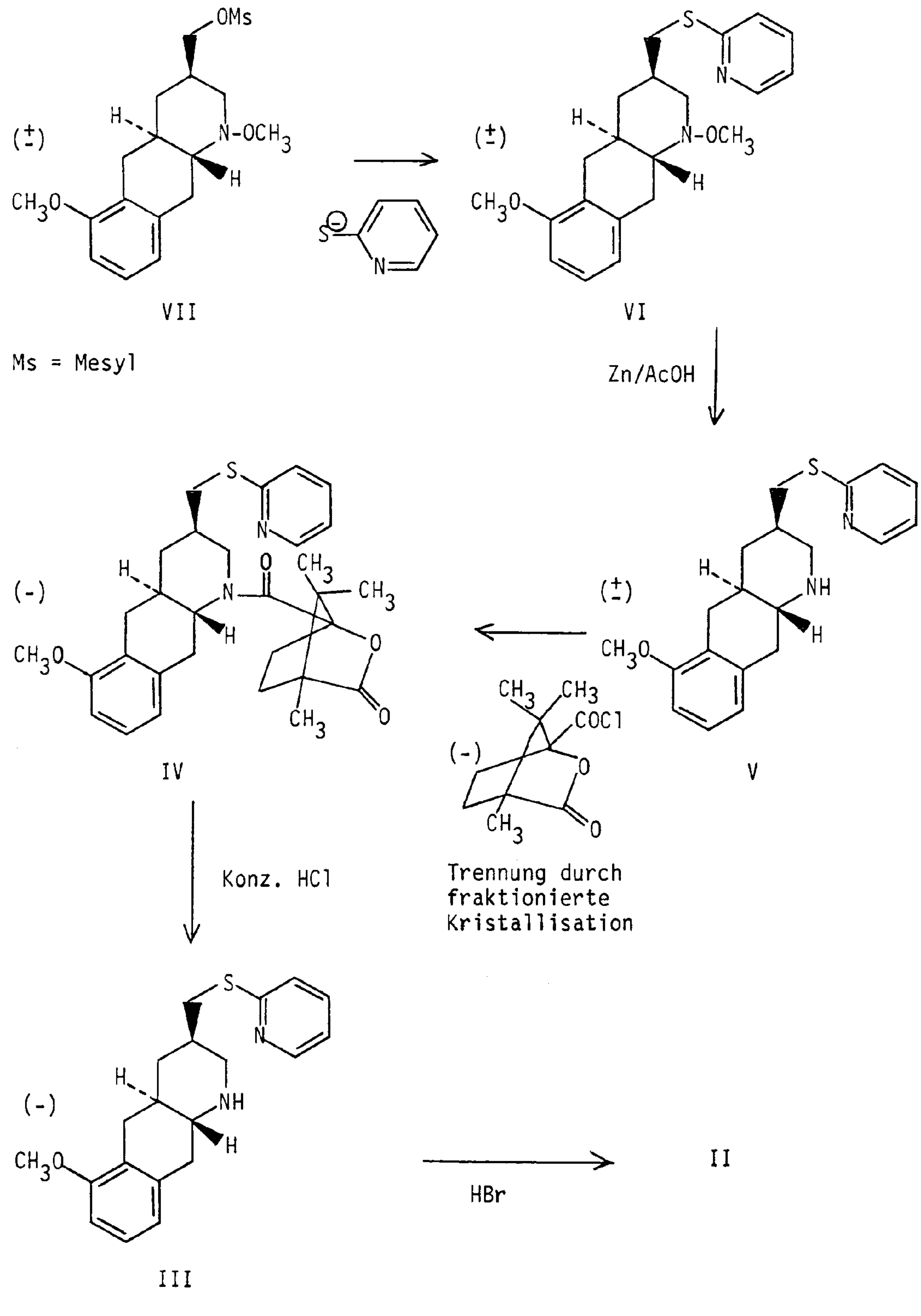


Die Ausgangsverbindung der Formel VII ist aus der Literatur bekannt.

Die Verbindung der Formel I und ihre physiologisch verträglichen Säureadditionssalze, im folgenden als erfindungsgemässe Verbindungen bezeichnet, zeichnen sich durch pharmakologische Wirkung aus und sind

deshalb verwendbar als Pharmazeutika.

Die erfindungsgemässen Verbindungen zeigen besonders eine dopaminerge Aktivität in vivo am zentralen Nervensystem, was nachgewiesen wird durch eine kontralaterale Rotation beim Verabreichen in Dosen von 1 bis 20 mg/kg p.o. oder 0,3 mg/kg s.c. an Ratten, bei denen durch eine 6-Hydroxydopamin-Injektion eine unilaterale Verletzung der nigrostriatalen Dopaminbahn verursacht wurde [U. Ungerstedt, Acta physiol. scand. Suppl. 367, 69 - 93 (1973)].

Die erfindungsgemässen Verbindungen sind deshalb brauchbar als dopaminerge Wirkstoffe, z.B. zur Behandlung von Morbus Parkinson.

Ferner bewirken die erfindungsgemässen Verbindungen eine Abnahme des intraokularen Drucks beim Kaninchen, mit Konzentrationen von 10 bis 100 μM. Männliche Kaninchen von ca. 2,5 kg werden in Käfige, die den Kopf freilassen, fixiert. Die Lösungen mit der zu prüfenden Verbindung werden in das rechte Auge und die Plazebo-Lösungen in das linke Auge appliziert (je 2 Tropfen, d.h. ca. 40 μl). Die Augen werden zuerst mit einer Lösung enthaltend Novensin (0,4 %) und Fluorescein (0,05 %) anästhesiert und der okulare Druck wird zu verschiedenen Zeitpunkten nach der Verabreichung (10, 20, 30, 60, 90, 120, 180 und 240 Minuten) bestimmt, wobei ein Applanations-Tonometer nach Goldberg verwendet wird.

Die erfindungsgemässen Verbindungen sind deshalb brauchbar zur Behandlung des Glaukoms.

Ueberdies bewirken die erfindungsgemässen Verbindungen bei der Maus eine Abnahme der Unbeweglichkeitszeit im Verzweiflungsversuch nach Porsolt [behavioural despair test, R.D. Porsolt et al., Arch. Int. Pharmacodyn., 229, 327 - 336 (1977)] nach Verabreichung von ca. 0,01 bis 10 mg/kg s.c.

Die erfindungsgemässen Verbindungen sind deshalb brauchbar als Antidepressiva.

Ferner bewirken die erfindungsgemässen Verbindungen eine Hemmung der Abhängigkeit von Kokain beim Verabreichen in Dosen von 0,1 bis 10 mg/kg p.o. an Affen, die sich die Droge gemäss der in Psychopharmacologia (Berl.) 16, 30 - 48 (1969) beschriebenen Methode selbst verabreichen.

Die erfindungsgemässen Verbindungen sind deshalb brauchbar zur Vorbeugung, Verminderung oder Behandlung einer Abhängigkeit (oder zur Behandlung nach dem Entzug zur Vermeidung erneuter Abhängigkeit), die bei Missbrauch von Kokain entsteht.

Für diese therapeutischen Wirkungen liegt eine geeignete tägliche Dosis im Bereich von ca. 1 bis 100 mg, insbesondere ca. 10 bis 80 mg, der erfindungsgemässen Verbindung. Geeignete Dosierungsformen für z.B. orale Anwendungen enthalten im allgemeinen ungefähr 0,25 bis 50 mg wirksame Substanz neben festen oder flüssigen Trägersubstanzen oder Verdünnungsmitteln.

Die erfindungsgemässen Verbindungen können auf jedem üblichen Weg verabreicht werden, vorzugsweise oral, beispielsweise in Form von Tabletten oder Kapseln, oder parenteral, beispielsweise in Form von Injektionslösungen oder Suspensionen.

Für die Behandlung des Glaukoms werden die erfindungsgemässen Verbindungen vorzugsweise in ca. 0,002 bis ca. 0,02 %-igen ophtalmologischen Lösungen ins Auge topisch appliziert.

Die vorliegende Erfindung betrifft ebenfalls pharmazeutische Zusammensetzungen, die eine erfindungsgemässe Verbindung zusammen mit zumindest einem pharmazeutischen Träger oder Verdünnungsmittel enthalten. Solche Zusammensetzungen können auf an sich bekannte Weise hergestellt werden.

Das nachfolgende Beispiel erläutert die Erfindung. Temperaturangaben erfolgen in Celsiusgraden und sind unkorrigiert.

**BEISPEIL: (-)-(3β,4aα,10aβ)-1,2,3,4,4a,5,10,10a-Octahydro-3-[(2-pyridylthio)methyl]-1-methyl-6-hydroxy-benzo[g]chinolin**

**a)(±)-(3β,4aα,10aβ)-1,2,3,4,4a,5,10,10a-Octahydro-3-[(2-pyridylthio)methyl]-1,6-dimethoxy-benzo[g]chinolin**

Eine Lösung von 11 g (30,9 mM) (±)-(3β,4aα,10aβ)-1,2,3,4,4a,5,10,10a-Octahydro-3-mesyloxymethyl-1,6-dimethoxy-benzot[g]chinolin und 11 g (100 mM) 2-Mercaptopyridin in 200 ml Dimethylformamid wird bei 10 - 15 ° mit 41 ml 2 N NAOH versetzt und bei Raumtemperatur 20 Stunden gerührt. Zur Aufarbeitung wird die Suspension am Rotationsverdampfer eingeengt, mit Wasser versetzt und mit Methylenchlorid extrahiert. Die organische Phasen werden über $Na_2SO_4$ getrocknet, filtriert und eingeengt. Nach Chromatographie an Kieselgel mit Methylenchlorid/3 % Ethanol resultieren 5,6 g der Titelverbindung (49 % der Theorie).

NMR ($CDCl_3$, 360 MHz) δ 0,94 (q, J = 12 Hz, H-$C9_{ax}$), 2,74 (dd, $J_1$ = 12 Hz, $J_2$ = 18 Hz, H-$C5_{ax}$), 3,61 (s, N-$OCH_3$), 3,8 (s, C-$OCH_3$).

**b) (±)-(3β,4aα,10aβ)-1,2,3,4,4a,5,10,10a-Octahydro-3-[(2-pyridylthio)methyl]-6-methoxy-benzo[g]chinolin**

10 g (27 mM) der unter a) erhaltenen Verbindung und 37,8 g (570 mM) Zink werden in einer Lösung von 55 ml Wasser und 110 ml Essigsäure suspendiert und bei Zimmertemperatur 20 Stunden gerührt. Die Suspension wird durch Hyflo filtriert, eingeengt, mit 2 N NaOH auf pH 7 - 8 gestellt und mit Methylenchlorid extrahiert. Nach Trocknung über $Na_2SO_4$, Filtration und Eindampfen am Rotationsverdampfer resultieren 8 g Rohprodukt als gelbes Oel, die an Kieselgel mit Methylenchlorid/2,5 % Methanol chromatographiert werden: Damit erhält man 4,6 g der Titelverbindung (50 % der Theorie) als gelbliches Oel.

NMR ($CDCl_3$, 360 MHz) δ 1,03 (q, J = 12 Hz, H-$C9_{ax}$), 2,67 (t, J = 12, H-$C5_{ax}$), 2,88 (b, N-H), 3,8 (s, C-$OCH_3$).

**c)(-)-(3β,4aα,10aβ)-1,2,3,4,4a,5,10,10a-Octahydro-3-[(2-pyridylthio)methyl]-6-methoxy-benzo[g]chinolin**

Zur Racematspaltung wird das unter b) erhaltene, racemische sec. Amin mit (-)-Camphansäurechlorid in die beiden diastereomeren Amide überführt, die durch fraktionierte Kristallisation aus Diisopropyläther getrennt werden. Die beiden diastereomeren Amide besitzen die folgenden Drehwerte ($CHCl_3$): $[\alpha]_D^{20}$ = 108° resp. $[\alpha]_D^{20}$ = + 86 °. Zur Amidhydrolyse werden 3,4 g (6,5 mM) des Amides mit $[\alpha]_D^{20}$= - 108 ° in 135 ml conc. HCl 16 Stunden bei 105° gerührt, mit Wasser verdünnt, bei 10 ° mit 10 N NaOH neutralisiert und mit Methylenchlorid/10 % Isopropanol extrahiert. Die organischen Phasen werden mit $Na_2SO_4$ getrocknet, filtriert und am Rotationsverdampfer eingedampft. Es resultieren 2 g Rohprodukt, die an Kieselgel mit Methylenchlorid/7 % Methanol chromatographiert werden. Die anfallenden 1,6 g des reinen Enantiomeren werden ohne Charakterisierung weiter verwendet.

**d) (-)-(3β,4aα,10aβ)-1,2,3,4,4a,5,10,10a-Octahydro-3-[(2-pyridylthio)methyl]-6-hydroxy-benzo[g]chinolin**

1,6 g (4,7 mM) des unter c) erhaltenen reinen Enantiomeren in 14 ml HBr (47 %ig) werden 6 Stunden bei 100 ° gerührt. Anschliessend wird eingeengt, mit 2 N NAOH neutralisiert und mit Methylenchlorid/10 % Isopropanol extrahiert. Nach Trocknung über $Na_2SO_4$, Filtration und Eindampfen resultieren 1,8 g rohes Hydroxyderivat, die mit Methylenchlorid/7 % Methanol an Kieselgel chromatographiert werden. Die anfallenden 1 g des reinen Hydroxyderivates werden sofort N-methyliert.

**e) (-)-(3β,4aα,10aβ)-1,2,3,4,4a,5,10,10a-Octahydro-3-[(2-pyridylthio)methyl]-1-methyl-6-hydroxy-benzo[g]chinolin**

Eine Lösung von 1 g (3,06 mM) des unter d) erhaltenen Hydroxyderivates und 8 ml wässerige 35 %ige Formaldehydlösung in 80 ml Methanol wird 30 Minuten bei Raumtemperatur gerührt und anschliessend bei 0 ° langsam portionenweise mit 2,2 g $NaBH_4$ versetzt. Nach 2 Stunden Rühren bei Raumtemperatur wird eingeengt, mit $NaHCO_3$-Lösung versetzt und mit Chloroform extrahiert. Trocknen über $Na_2SO_4$, Filtration und Eindampfen liefern 1 g (99 % der Theorie) reine Titelverbindung, die aus Aceton/Essigester kristallisieren: $[\alpha]_D^{20}$ = - 162° (Pyridin). Nach Umkristallisation aus Aceton/Essigester: $[\alpha]_D^{20}$ = - 160° (Pyridin). Smp. = 186 - 187 °.

## Patentansprüche

1. Das (-)-(3β,4aα,10aβ)-1,2,3,4,4a,5,10,10a-Octahydro-3-[(2-pyridylthio)methyl]-1-methyl-6-hydroxy-benzo[g]chinolin der Formel I

(-) 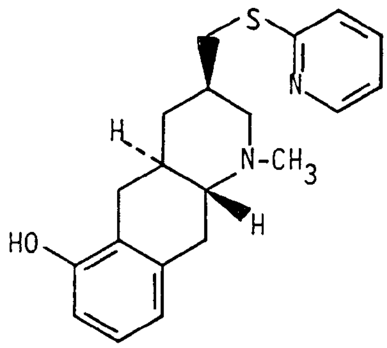 (I)

in Form der freien Base oder in Säureadditionssalzform.

2. Die Verbindung der Formel I gemäss Anspruch 1 in Form der freien Base.

3. Die Verbindung der Formel I gemäss Anspruch 1 in Form des Hydrochlorids.

4. Verfahren zur Herstellung einer Verbindung gemäss Anspruch 1, dadurch gekennzeichnet, dass man die Verbindung der Formel II

(-) 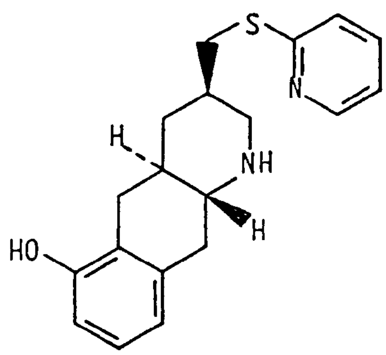 (II)

methyliert und die erhaltene Verbindung gewünschtenfalls in ihre Säureadditionssalze überführt.

5. Eine Verbindung gemäss einem der Ansprüche 1 bis 3, als freie Base oder in physiologisch verträglicher Säureadditionssalzform, zur Anwendung als Pharmazeutikum.

6. Eine Verbindung gemäss einem der Ansprüche 1 bis 3, als freie Base oder in physiologisch verträglicher Säureadditionssalzform, zur Behandlung von Morbus Parkinson, der Depression oder der Kokain-Abhängigkeit.

7. Eine Verbindung gemäss einem der Ansprüche 1 bis 3, als freie Base oder in physiologisch verträglicher Säureadditionssalzform, zur Behandlung des Glaukoms.

8. Eine pharmazeutische Zusammensetzung, enthaltend eine Verbindung gemäss einem der Ansprüche 1 bis 3, als freie Base oder in physiologisch verträglicher Säureadditionssalzform.

9. Die Anwendung einer Verbindung gemäss einem der Ansprüche 1 bis 3, für die Herstellung einer pharmazeutischen Zusammensetzung zur Behandlung von Morbus Parkinson, der Depression oder der Kokain-Abhängigkeit.

10. Die Anwendung einer Verbindung gemäss einem der Ansprüche 1 bis 3, für die Herstellung einer pharmazeutischen Zusammensetzung zur Behandlung des Glaukoms.

**Patentansprüche für folgende Vertragsstaaten : ES, GR**

1. Verfahren zur Herstellung des (-)-(3β,4aα,10aβ)-1,2,3,4,4a,5,10,10a-Octahydro-3-[(2-pyridylthio)methyl]-1-methyl-6-hydroxy-benzo[g]chinolins der Formel I

(-) 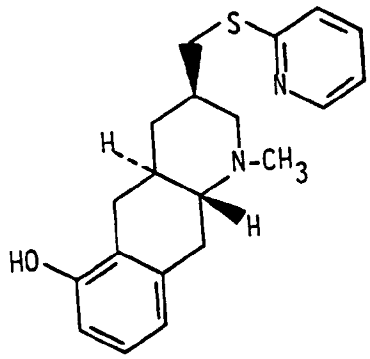 (I)

und seiner Säureadditionssalze, dadurch gekennzeichnet, dass man die Verbindung der Formel II

(-) 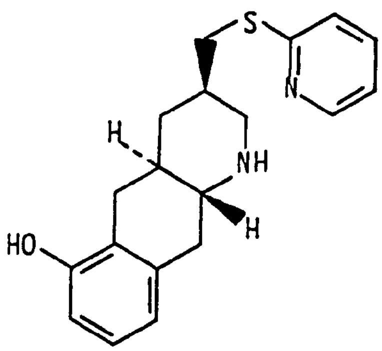 (II)

methyliert und die erhaltene Verbindung gewünschtenfalls in ihre Säureadditionssalze überführt.

2. Verfahren gemäss Anspruch 1, dadurch gekennzeichnet, dass man die Verbindung der Formel I in Form der freien Base herstellt.

3. Verfahren gemäss Anspruch 1, dadurch gekennzeichnet, dass man die Verbindung der Formel I in Form des Hydrochlorids herstellt.

4. Verfahren zur Herstellung einer pharmazeutischen Zusammensetzung, enthaltend eine Verbindung der Formel I gemäss Anspruch 1 in Form der freien Base oder eines physiologisch verträglichen Säureadditionssalzes, dadurch gekennzeichnet, dass man die Verbindung der Formel I mit einem pharmazeutischen Träger oder Verdünnungsmittel vermischt.

Europäisches Patentamt

# EUROPÄISCHER TEILRECHERCHENBERICHT

der nach Regel 45 des Europäischen Patentübereinkommens für das weitere Verfahren als europäischer Recherchenbericht gilt

Nummer der Anmeldung
EP 92 81 0306

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int. Cl.5) |
|---|---|---|---|
| D,X | EP-A-0 077 754 (SANDOZ-PATENT GmbH)<br>* Ansprüche 1-8, 14-16; Seiten 39-43 *<br>--- | 1,5-10 | C 07 D 401/12<br>A 61 K 31/44 |
| A | GB-A-2 134 515 (SANDOZ LTD)<br>* Ansprüche; Seiten 6,7 *<br>----- | 1,5-10 | |
| | | | RECHERCHIERTE SACHGEBIETE (Int. Cl.5) |
| | | | C 07 D |

## UNVOLLSTÄNDIGE RECHERCHE

**Nach Auffassung der Recherchenabteilung entspricht die vorliegende europäische Patentanmeldung den Vorschriften des Europäischen patentübereinkommens so wenig, daß es nicht möglich ist, auf der Grundlage einiger Patentansprüche sinnvolle Ermittlungen über den Stand der Technik durchzuführen.**
**Vollständig recherchierte Patentansprüche:**
**Unvollständig recherchierte Patentansprüche:**
**Nicht recherchierte Patentansprüche:**
**Grund für die Beschränkung der Recherche:**

Bemerkung: Obwohl die Ansprüche 6-7 sich auf ein Verfahren zur Behandlung des menschlichen Körpers beziehen (Art. 52(4) EPU) wurde die Recherche durchgeführt und gründete sich auf die angeführten Wirkungen der Verbindung.

| Recherchenort | Abschlußdatum der Recherche | Prüfer |
|---|---|---|
| DEN HAAG | 30-07-1992 | HENRY J.C. |

**KATEGORIE DER GENANNTEN DOKUMENTEN**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0409)